# EUROPÄISCHE PATENTSCHRIFT

(11) **EP 2 185 710 B1**
(45) Veröffentlichungstag und Bekanntmachung des Hinweises auf die Patenterteilung: **21.03.2012**
(21) Anmeldenummer: 08786675.2
(22) Anmeldetag: 31.07.2008
(51) Int. Cl.: C12N 15/81, C12P 21/02, C12N 5/10, C12N 1/16

(54) **EUKARYOTISCHE PROMOTOREN ZUR GENEXPRESSION**
EUKARYOTIC PROMOTERS FOR GENE EXPRESSION
PROMOTEURS EUCARYOTES POUR L'EXPRESSION GÉNIQUE

(30) Priorität: 10.08.2007 DE 102007038037
(43) Veröffentlichungstag der Anmeldung: 19.05.2010
(73) Patentinhaber: Leibniz-Institut für Pflanzengenetik und Kulturpflanzenforschung (IPK), 06466 Gatersleben (DE)
(72) Erfinder: BÖER, Erik, 06484 Quedlinburg (DE); KUNZE, Gotthard, 06466 Gatersleben (DE)
(74) Vertreter: Taruttis, Stefan Georg
(86) Internationale Anmeldenummer: PCT/EP2008/060050
(87) Internationale Veröffentlichungsnummer: WO 2009/021853

(56) Entgegenhaltungen:
- WO-A-01/85925
- DE-A1-102004 032 216
- SIVERIO J M ED - GELLISSEN G: "CHAPTER 3: Biochemistry and genetics of nitrate assimilation" HANSENULA POLYMORPHA: BIOLOGY AND APPLICATIONS, WILEY-VCH VERLAG, 1. Januar 2002 (2002-01-01), Seiten 21-40, XP001248616 ISBN: 978-3-527-30341-0
- GARCIA-LUGO PABLO ET AL: "Cloning, sequencing, and expression of H.a.YNR1 and H.a.YNI1, encoding nitrate and nitrite reductases in the yeast Hansenula anomala" YEAST, Bd. 16, Nr. 12, 15. September 2000 (2000-09-15), Seiten 1099-1105, XP002504711 ISSN: 0749-503X
- DIJK VAN R ET AL: "THE METHYLOTROPHIC YEAST HANSENULA POLYMORPHA: A VERSATILE CELL FACTORY" ENZYME AND MICROBIAL TECHNOLOGY, STONEHAM, MA, US, Bd. 26, 1. Juni 2000 (2000-06-01), Seiten 793-800, XP000994959 ISSN: 0141-0229
- GELLISSEN ET AL: "New yeast expression platforms based on methylotrophic Hansenula polymorpha and Pichia pastoris and on dimorphic Arxula adeninivorans and Yarrowia lipolytica - A comparison" FEMS YEAST RESEARCH, ELSEVIER SCIENCE, TOKYO, NL, Bd. 5, Nr. 11, 1. November 2005 (2005-11-01), Seiten 1079-1096, XP005162218 ISSN: 1567-1356
- STEINBORN ET AL: "Construction of an Arxula adeninivorans host-vector system based on trp1 complementation" JOURNAL OF BIOTECHNOLOGY, ELSEVIER SCIENCE PUBLISHERS, AMSTERDAM, NL, Bd. 127, Nr. 3, 1. Dezember 2006 (2006-12-01), Seiten 392-401, XP005787031 ISSN: 0168-1656 in der Anmeldung erwähnt
- DATABASE EMBL [Online] 8. Juli 2008 (2008-07-08), KUNZE, G.: "Arxula adeninivorans gene cluster for nitrate assimilation, strain LS3" XP002504712 gefunden im EBI accession no. EMBL:FM179611 Database accession no. FM179611

## Beschreibung

Die vorliegende Erfindung betrifft eukaryotische Promotoren, insbesondere induzierbare Promotoren zur Verwendung in Expressionskassetten, beispielsweise zur Verwendung bei der homologen oder heterologen Expression von Genen in eukaryotischen Zellen, vorzugsweise in Hefe.

Promotoren aus Hefen sind z.B. aus der EP 0599971 bekannt, die unter anderem Promotoren der Alkoholdehydrogenase aus *Kluyveromyces* und *Saccharomyces cerevisiae* beschreibt.

Die WO 01/85925 A2 beschreibt die Proteinexpression in *Arxula* mit Expressionskassetten, die unter anderem einen konstitutiven Promotor des *ARFC3-* oder des *AHSB4-,* des *AACP10 -* des *APCR7-* oder des *APRE4*-Gens enthalten können.

Bekannte induzierbare Promotoren zur Verwendung bei der Genexpression in Hefe weisen den Nachteil auf, dass zur Induktion ein kostenintensives Substrat erforderlich ist, beispielsweise Maltose, Xylitol oder Sucrose für den *GAA-, AXDH-* oder den *AINV-*Promotor, oder die durch eine erhöhte Konzentration an Natriumchlorid induzierbar sind, wie beispielsweise der *AHOG-* und der AORF104-Promotor.Siverio in "Hansenula polymorpha: Biology and Applications" Hersg. Gellissen, S. 21-40 (2002) beschreibt die Expression von Fusionsproteinen, die aus der Nitratreduktase, der Nitritreduktase und des Nitrattransporters aus Hansenula polymorpha und lacZ bestehen und unter der Kontrolle ihrer homologen Promotoren bei Induktion mit Nitrit oder Nitrat exprimiert werden. Garcia-Lugo et al, Yeast 1099-1105 (2000) beschreiben die Expression einer Nitritreduktase und einer Nitratreduktase in Hansenula bei Induktion mit Nitrit und Nitrat.

### Aufgabe der Erfindung

Aufgabe der vorliegenden Erfindung ist, alternative Promotoren bereitzustellen, mit denen in Eukaryonten, beispielsweise in Hefen, vorzugsweise in *Arxula,* die Expression von funktional zu den Promotoren angeordneten Genen erfolgen kann. Vorzugsweise sind erfindungsgemäße Promotoren mit einem kostengünstigen Substrat induzierbar. Besonders bevorzugt können mit den erfindungsgemäßen Promotoren versehene Expressionskassetten zur Produktion von Translationsprodukten verwendet werden, die mit bisher verfügbaren Promotoren nicht oder nur in schlechter Ausbeute zu erzeugen waren.

### Allgemeine Beschreibung der Erfindung

Die Erfindung löst die vorgenannte Aufgabe mit den Merkmalen von Anspruch 1 mit Promotoren, die in Hefe der Gattung *Arxula,* insbesondere *Arxula adeninivorans* identifizierbar sind. Die Promotoren sind als Sequenzen *AYNR1* (Seq.-ID Nr. 1), *AYNR1'* (Seq.-ID Nr. 4), *AYNI1* (Seq.-ID Nr. 2), *AYNI1'* (Seq.-ID Nr. 5) und *AYNT1* (Seq.-ID Nr. 3), *AYNT1'* (Seq.-ID Nr. 6) enthalten und stammen von Genen der Nitratreduktase (*AYNR1*), Nitritreduktase (*AYNI1*) bzw. des Nitrattransporters (*AYNT1*) aus *Arxula adeninivorans.* Entsprechend sind Promotoren mit Nitrat und/oder Nitrit induzierbar in der Gruppe von Sequenzen enthalten, die aus den Seq.-ID Nrn. 1 bis 6 besteht.

Die Promotoren sind zur Verwendung in Expressionskassetten geeignet, um ein Strukturgen, das funktional zu dem Promotor angeordnet ist, zu transkribieren und zu translatieren.

Weiterhin stellt die Erfindung das Verfahren mit Abschnitten der Promotoren der Sequenzen mit den Seq.-ID Nrn. 1, 2 und 3 mit Promotoraktivität bereit, vorzugsweise mit einer Länge von mindestens 50 %, sowie Derivate der Seq.-ID Nm. 1, 2 und 3 mit Promotoraktivität, beispielsweise mit Sequenzabweichungen in 1 bis 100 bp, vorzugsweise bis maximal 5%, besonders bevorzugt bis maximal 1% Sequenzabweichung. Besonders bevorzugt sind die Promotoren mit Nitrat (NO₃⁻) und/oder Nitrit (NO₂⁻) induzierbar.

Die Erfindung stellt Verfahren zur Expression von Proteinen in Arxula bereit, wobei zur intrazellulären oder extrazellulären Expression Expressionskassetten mit einem gewünschten Strukturgen verwendet werden, das funktional zu einem erfmdungsgemäßen Promotor angeordnet ist.

Eukaryotische Zellen, in denen die erfindungsgemäßen Promotoren durch genetische Manipulation enthalten sind, sind sind *Arxula.*

### Genaue Beschreibung der Erfindung

Die Promotoren der Sequenzen Seq.-ID Nr. 1, Seq.-ID Nr. 2, Seq.-ID Nr. 3, Seq.-ID Nr. 4, Seq.-ID Nr. 5 und Seq.-ID Nr. 6, daraus herstellbare Derivate mit einer Sequenzhomologie von mindestens 80%, bevorzugt mindestens 90%, besonders bevorzugt mindestens 95% und/oder mit oder aus Nukleinsäuresequenzen, die unter normalen, vorzugsweise unter stringenten Bedingungen mit einer der Seq.-ID Nr. 1 bis 6 hybridisieren, sowie Promotoren, die Abschnitte der Seq.-ID Nr. 1, Seq.-ID Nr. 2 oder Seq.-ID Nr. 3 aufweisen, vorzugsweise mit einer Länge von mindestens 50%, mindestens 75%, mindestens 80%, besonders bevorzugt mindestens 90%, sind geeignet, in Expressionskassetten für homologe oder heterologe Gene in eukaryotischen Zellen verwendet zu werden, um die Transkription der funktional zu den Promotoren angeordneten Strukturgene zu kontrollieren, gegebenenfalls in Verbindung mit deren Translation. Die Sequenzen Seq.-ID Nrn. 4, 5 und 6 sind Beispiele für Abschnitte mit Promotoraktivität der Sequenzen Seq.-ID Nrn. 1, 2 bzw. 3. Weitere DNA-Abschnitte, die auch Derivate mit hoher Sequenzhomologie sein können, sind dadurch identifizierbar, dass verkürzte und/oder zu maximal 20 %, bevorzugt zu maximal 10 oder 5 % der Basensequenz derivatisierte Abschnitte der Promotoren der Seq.-ID Nrn. 1 bis 6 hergestellt und in Expressionskassetten, z.B. wie in den vorliegenden Beispielen dargestellt, kloniert und auf Promotoraktivität und Induzierbarkeit getestet werden. Die erfindungsgemäßen Promotoren sind vorzugsweise mit NO₃⁻ induzierbar, das z.B. im Kulturmedium vorliegen kann.

Derivate und Abschnitte der Seq.-ID Nrn. 1 bis 6 sind in einer der beispielhaft dargestellten Expressionskassetten als Promotor zu identifizieren, wenn sie mit Nitrat und/oder Nitrit induzierbar sind, und insbesondere die Expression eines Strukturgens in *Arxula* erlauben, das mit einem herkömmlichen Promotor nicht oder mit geringerer Ausbeute an aktivem Protein exprimiert wird. Eine bevorzugte Expressionskassette zum Testen erfindungsgemäßer Derivate und Abschnitte der Seq.-ID Nrn. 1, 2 und 3 ist in Beispiel 5 mit dem *Tannase-Gen* als Strukturgen, in 3' gefolgt vom *PHO5*-Terminator beschrieben, wobei der TEF1-Promotor als Vergleich eingesetzt ist.

Das enthaltene Sequenzprotokoll zeigt die Sequenzen in 5' nach 3' Richtung, so dass die unter der Kontrolle eines erfindungsgemäßen Promotors zu transkribierende DNA-Sequenz am 3'-Ende des Promotors angeordnet werden kann. Vorzugsweise ist das Startkodon einer zu transkribierenden Sequenz unmittelbar oder in einem Abstand von bis zu 30 bp, bevorzugter bis 15 bp vom 3'-Ende des Promotors entfernt angeordnet.

Besonders bevorzugt weisen Expressionskassetten einen der Promotoren, eine in 3' angeordnete transkribierbare, vorzugsweise auch translatierbare DNA-Sequenz, und weiter in 3' zur transkribierbaren DNA-Sequenz einen Terminator auf.

Besonders bevorzugt sind erfindungsgemäße Expressionskassetten einseitig, vorzugsweise beidseitig von Sequenzabschnitten flankiert, die eine Homologie von mindestens 80% zu dem Wirtsorganismus aufweisen, um in dessen chromosomale DNA integriert zu werden.

Für die Zwecke dieser Erfindung umfasst der Begriff einer Expressionskassette sowohl DNA-Konstrukte, die eine zur Transkription geeignete transkribierbare DNA-Sequenz in funktionaler Anordnung aufweisen, als auch zur Proteinproduktion, d.h. zur Translation geeignete transkribier- und translatierbare Sequenzen, jeweils funktional zum Promotor angeordnet.

Weiter bevorzugt weisen erfindungsgemäß verwendete Expressionskassetten einen mit diesen verbundenen DNA-Abschnitt auf, der zur Selektion geeignet ist, beispielsweise einen exprimierbaren Selektionsmarker in einer Expressionskassette, vorzugsweise unter der Kontrolle eines anderen, konstitutiven oder induzierbaren Promotors, als er in der Expressionskassette mit erfindungsgemäβ verwendetem Promotor enthalten ist.

Ein- oder beidseitig die erfindungsgemäß verwendete Expressionskassette flankierend angeordnet sind vorzugsweise homologe Sequenzen, die eine ein- oder mehrfache Integration der Expressionskassette in das Genom der Wirtszelle ermöglichen. Die Homologie dieser flankierenden Sequenzen beträgt vorzugsweise mindestens 80% zur rDNA des Zielorganismus.

Für die mehrfache Integration erfindungsgemäß verwendeter Expressionskassetten ist es bevorzugt, dass diese mit einer Expressionskassette für ein Selektionsmarkergen verbunden sind, wobei das Selektionsmarkergen eine Auxotrophie des Zielorganismus komplementierendes Strukturgen unter der Kontrolle eines Promotors aufweist, der eine auf maximal 20% der Aktivität des ursprünglichen Promotors reduzierte Aktivität dieses Strukturgens aufweist.

Wirtsorganismen zur Genexpression sind Hefen der Gattung *Arxula,* insbesondere *Arxula adeninivorans,* die durch genetische Manipulation eine Expressionskassette mit einem nitratinduzierbaren Promotor aufweisen. Denn Hefen der Gattung *Arxula* können NO₃⁻ als Stickstoffquelle nutzen, sodass NO₃⁻ neben der Wirkung als Induktor für die erfindungsgemäße Expressionskassette auch eine Stickstoffquelle bei Fermentationen ist. Damit stellt die Erfindung ein Verfahren mit Promotoren bereit, die mit einem kostengünstigen Substrat induzierbar sind, das überdies bei Verwendung von Zielorganismen, die NO₃⁻ verstoffwechseln können, beispielsweise als Stickstoffquelle genutzt werden kann.

Die erfindungsgemäß verwendeten Zielorganismen der Gattung *Arxula* für die genetische Manipulation mit einer Expressionskassette, die einen erfindungsgemäß verwendeten Promotor aufweist, haben überdies den Vorteil, dass Translationsprodukte im Anschluss an die Translation zumindest im wesentlichen nicht degradiert werden.

Die Expressionskassetten sind überdies geeignet, Translationsprodukte von Genen durch heterologe Genexpression in Wirtsorganismen herstellen zu können, die mit Expressionskassetten herkömmlicher Promotoren nicht oder nur in geringen Konzentrationen herstellbar sind, oder im Anschluss an die Translation degradiert werden. Denn die erfindungsgemäß verwendeten Promotoren ermöglichen auch die Genexpression komplexer oder empfindlicher Translationsprodukte.

Die Erfindung wird nun genauer anhand von Beispielen mit Bezug auf die Figuren erläutert, in denen
- Figur 1 eine schematische Darstellung eines Shuttle-Vektors mit erfindungsgemäß verwendeter Expressionskassette zeigt,
- Figur 2 unter A das Wachstumsverhalten von erfindungsgemäß verwendeten (■, *, ▲, ●) und Vergleichs-Transformanden (◆, x) und unter B die von den Transformanden produzierte extrazelluläre Aktivität von Phytase K zeigt,
- Figur 3 unter A die Induktion der Translation durch den erfindungsgemäß verwendeten Promotor für Transformanden (■, *, ▲, ●) und einen Kontroll-Transformand (◆, x) im Shift-Experiment von NH₄⁺-haltigem Medium in NH₄⁺-haltiges (x, *, ●) bzw. in NO₃⁻ -haltiges Medium (◆, ▲, ■) zeigt und unter B die Abhängigkeit der Translationsintensität erfindungsgemäβ verwendeter Transformanden (■, ▲) und des Vergleichs-Transformanden (◆) von der Induktorkonzentration nach Übertragung von NH₄⁺-haltigem Medium in NO₃⁻ -haltiges,
- Figuren 4 und 5 schematische Darstellungen von Shuttle-Vektoren mit erfindungsgemäß verwendeter Expressionskassette zeigen,
- Figur 6 eine schematische Darstellung eines Shuttle-Vektors mit Expressionskassette mit *TEF1*-Promotor zeigt,
- Figur 7 einen Vergleich der Induktion der Proteinexpression von erfindungsgemäß verwendeten Expressionskassetten unter A, B und C ,gegenüber D der Expressionskassette mit *TEF1*-Promotor nach anfänglicher Kultivierung in NH₄⁺-haltigem Medium in NH₄⁺-haltigem Medium (◆,■) oder NO₃⁻ -haltigem Medium (x, ▲) zeigt,
- Figur 8 eine schematische Darstellung eines Shuttle-Vektors mit erfindungsgemäß verwendeter Expressionskassette zur Expression von Tannase zeigt,
- Figur 9 unter A die Wachstumsraten von Tranformanden nach Figur 8 zeigt und unter B die Aktivität erfindungsgemäß verwendeter Transformanden in NO₃⁻ - haltigem Medium (□) oder NH₄⁺-haltigem Medium (■) und einer Negativkontrolle (◆ in NH₄⁺, 0 in NO₃⁻) an Tannase und
- Figur 10 einen Vergleich der Induktion der Proteinexpression der erfindungsgemäβ verwendeten Expressionskassette mit dem *Tannase-*Gen gegenüber der Kontrolle ohne Expressionskassette.

### Beispiel 1: Promotor auf Basis der Seq.-ID Nr. 1

Der Promotor mit der Seq.-ID Nr. 1 (*AYNR1*-Promotor) wurde per PCR mit Primern, die zusätzlich in 5' eine *Spe*I Restriktionsschnittstelle, und in 3' eine *Eco*RI Restriktionsschnittstelle aufwiesen, als 897 bp Fragment amplifiziert. Durch Ligation wurde in 3' an das Amplifikat mit der Seq.-ID Nr. 1 an die *Eco*RI Schnittstelle das *phyK*-Gen (kodiert Phytase von *Klebsiella* sp. ASR1 - Sajidan A, Farouk A, Greiner R, Jungblut P, Müller EC, Borriss R (2004) Appl. Microbiol. Biotechnol. 65: 110-118) angeordnet. In 3' zum *phyK-*Gen wurde der *PHO5*-Terminator angeordnet. Diese Expressionskassette wies nun als Strukturgen das *Phytase K*-Gen auf, das in 3' vom *PHO5-*Terminator begrenzt wurde. Dieses Konstrukt wurde mit einer Selektionsmarkerkassette versehen, nämlich dem *ATRP1*-Gen unter der Kontrolle des *ALEU2*-Promotors, in 3' flankiert vom *ATRP1*-Terminator. Das *ATRP1*-Gen komplementiert die entsprechende *atrp1* Mutation des für Tryptophan auxotrophen Stammes von *Arxula adeninivorans* G1212 [*aleu2 ALEU2::Δatrp1*] (Steinborn G, Wartmann T, Gellissen G, Kunze G (2007) J. Biotechnol. 127: 392-401), der als Zielorganismus verwendet wurde.

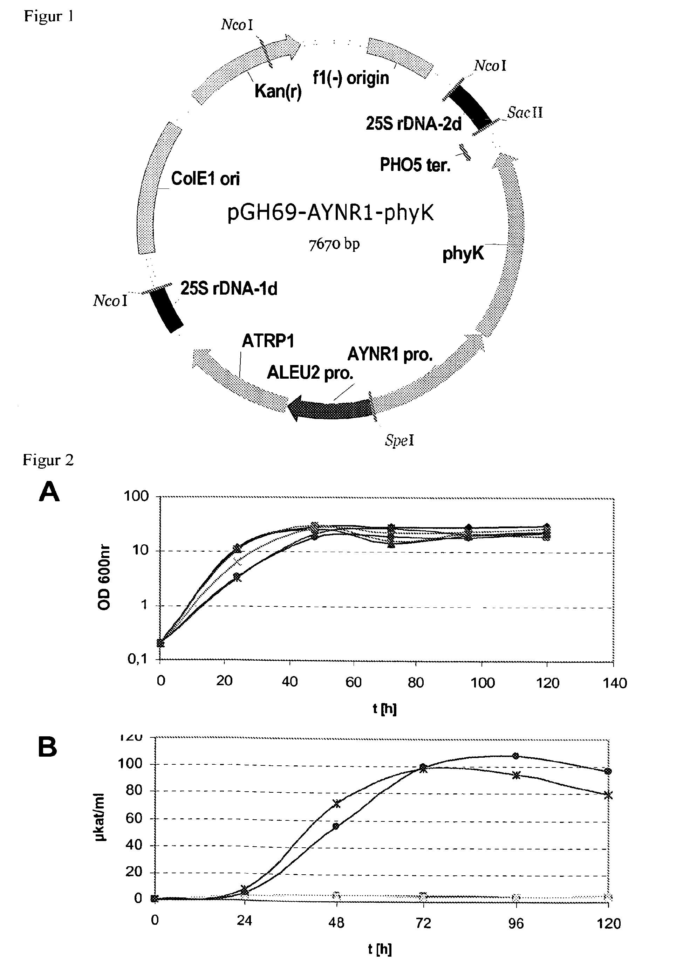

Die Expressionskassette und die Selektionsmarkerkassette wurden miteinander ligiert und zusammen beidseitig von Abschnitten, die der 25S rDNA des Zielorganismus entstammen, flankiert. Eine schematische Darstellung dieses DNA-Konstruktes in einem *E. coli* Shuttle-Vektor mit *E. coli* Plasmid-Teil und einer "Yeast Integration Expression Cassette" (YIEC) ist in Figur 1 gezeigt.

Zur Transformation von *Arxula* wurde die Expressionskassette, verbunden mit der Selektionsmarkerkassette, zusammen zur Integration beidseitig von homologen Sequenzabschnitten flankiert (YIEC), mittels der *Nco*I-Restriktionsschnittstellen isoliert oder über PCR amplifiziert und in *Arxula adeninivorans* G 1212 transformiert. Nach Selektion auf Hefe-Minimalmedium plus 2% Glukose (G-HMM) mit NH₄⁺ als Stickstoffquelle (Tanaka A, Ohnishi N, Fukui S (1967) J. Ferment. Technol. 45: 617-623) wurden Transformanden (G1212/YIEC69-AYNR1-phyK) erhalten, die das DNA Konstrukt in ihr Genom integriert aufwiesen, nämlich unikal in der 25S rDNA.

Zum Vergleich wurde *Arxula adeninivorans* G1212 mit einer YIEC transformiert, die mit Ausnahme der Expressionskassette für Phytase K identisch war. (Negativkontrolle *Arxula adeninivorans* G1212/YIEC69).

Für die Expression wurden die Transformanden für bis zu 120 h in G-HMM mit NH₄⁺ (43,5 mM (NH₄)₂SO₄) bzw. NO₃⁻ (43,5 mM NaNO₃) als Stickstoffquelle kultiviert. Während der Kultivierung wurde die Aktivität der rekombinanten Phytase K analysiert.

Die Ergebnisse sind in Fig. 2 B dargestellt und zeigen, dass in *A. adeninivorans* G1212/YIEC69-AYNR1-phyK kultiviert in G-HMM mit NO₃⁻(*, ●) die Phytase-Aktivität kontinuierlich während der ersten 72 h der Kultivierung ansteigt und in der stationären Wachstumsphase ein Maximum erreicht. Während der weiteren Kultivierung bis zu 120 h sinkt diese Aktivität nur geringfügig. Sowohl bei der mit dem erfindungsgemäßen DNA-Konstrukt transformierten Hefe (G1212/YIEC69-AYNR1-phyK, Klon 1-5: ■, *; Klon 1-6: ▲, ●), als auch bei dem Vergleichs-Transformand (G1212/YIEC69: ◆, x) wurde dieselbe Wachstumsrate gefunden (Figur 2 A). Phytase K dagegen wurde nur von erfindungsgemäßen Transformanden produziert.

Bei Kultivierung in G-HMM mit NH₄⁺ (■, ▲) wurde keine rekombinante Phytase K detektiert, weder intrazellulär noch extrazellulär. Auch der Kontroll-Transformand (G1212/YIEC69) wies während der Kultivierung keine Phytase-Aktivität auf.

Die Induktion des erfindungsgemäß verwendeten Promotors durch NO₃⁻ im G-HMM konnte auch durch NO₂⁻(43,5 mM NaNO₂) erzielt werden.

Die Induktion bei erfindungsgemäß verwendeten Transformanden konnte auch im sogenannten Shift-Experiment, d.h. nach Kultivierung unter nicht-induzierenden Bedingungen im NO₃⁻- und NO₂⁻-freien Medium und anschließende Übertragung in G-HMM mit NO₃⁻ oder NO₂⁻ erzielt werden. So konnten erfindungsgemäß verwendete Transformanden und Vergleichs- Transformanden nach Kultivierung in G-HMM mit NH₄⁺ über 48 h, während derer keine Phytase K produziert wurde, abzentrifugiert werden und in frisches G-HMM mit NH₄⁺ bzw. NO₃⁻ übertragen werden. Nur der erfindungsgemäße Transformand (G1212/YIEC69-AYNR1-phyK) produzierte im NO₃⁻-haltigen Medium rekombinante Phytase K, die extrazellulär nachgewiesen wurde. Im Anschluss an die Übertragung in NO₃⁻- bzw. NO₂⁻-haltiges Medium stieg die Aktivität der Phytase kontinuierlich an und erreichte nach etwa 30 h ihr Maximum. Während der weiteren Kultivierung blieb die Aktivität der Phytase K nahezu konstant, was zeigt, dass zumindest die einmal erreichte Konzentration enzymatisch aktiven Translationsprodukts der erfindungsgemäßen Expressionskassette nicht degradiert wird.

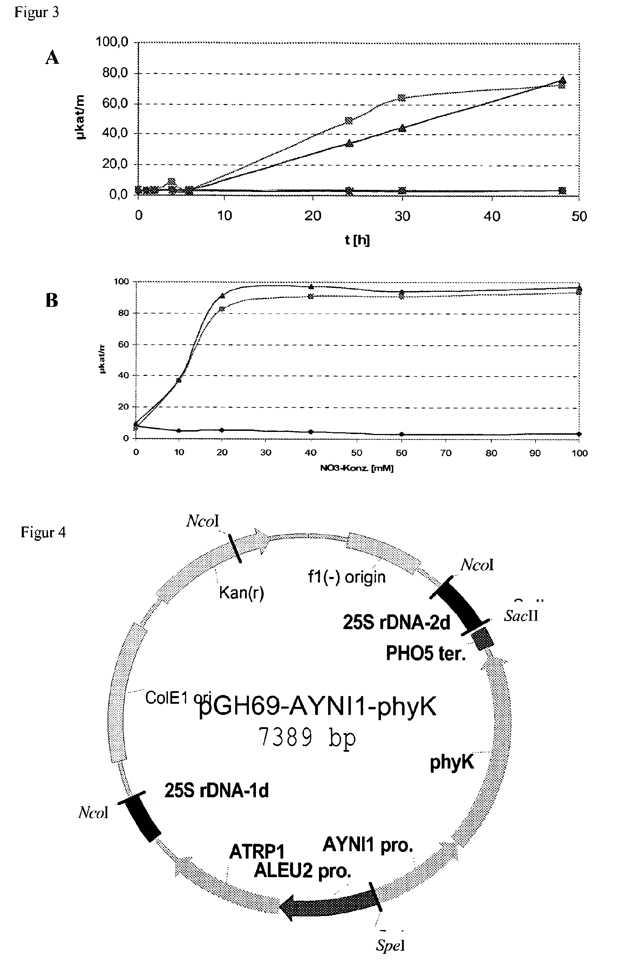

Bei Übertragung in frisches G-HMM mit NH₄⁺ wurde weder intrazellulär noch extrazellulär Phytase-Aktivität nachgewiesen. Die Analysenergebnisse für die Phytaseaktivität sind in Figur 3 A für den Kontroll-Transformand G1212/YIEC69 (◆, x) und den erfindungsgemäßen Transformanden G1212/YIEC69-AYNR1-phyK Klon 1-5 (*, ■**)** und Klon 1-6 (▲, ●) gezeigt, und im Anschluss an eine anfängliche Kultivierung für 48 h in G-HMM mit NH₄⁺ (30 °C), und nach der Übertragung in frisches G-HMM mit NH₄⁺ (x, *, ●) oder NO₃⁻ (◆, ■. ▲) während der Kultivierung für 48 h bei 30 °C.

Für die erfindungsgemäß verwendete Expressionskassette mit *Phytase K* als Beispiel für ein heterolog exprimiertes Strukturgen konnte gezeigt werden, dass die Intensität der Expression unter Kontrolle der erfindungsgemäßen Promotoren von der Konzentration des Induktors abhängig ist. Dazu wurde zur Kontrolle G1212/YIEC69 (◆) eingesetzt, und erfindungsgemäße Klone G1212/YIEC69-AYNR1-phyK 1-5 (■) und Klon 1-6 (A) für 48 h in G-HMM mit NH₄⁺ bei 30 °C kultiviert, abzentrifugiert und unmittelbar in neues G-HMM mit unterschiedlichen Konzentrationen an NO₃⁻ übertragen und für weitere 30 h bei 30 °C kultiviert. Anschließend wurden die Zellen abgetrennt und die extrazelluläre Phytase-Aktivität gemessen. Das Ergebnis ist in Figur 3 B dargestellt und zeigt, dass die Induktion bis zu einer Konzentration von ca. 20 mM NO₃⁻ von der Konzentration des Induktors abhängig war, während höhere Konzentrationen an Induktor die Translation nicht weiter steigern, jedoch auch nicht beeinträchtigen. Der Kontroll- Stamm synthetisierte unabhängig von den Kultivierungsbedingungen keine Phytase.

Bei Ersetzen des Promotors der Seq.-ID Nr. 1 durch die Sequenz Seq.-ID Nr. 7 wurden ähnliche Ergebnisse erzielt.

### Beispiel 2: Promotor auf Basis der Seq.-ID Nr. 2

Entsprechend Beispiel 1 wurden Expressionskassetten mit dem Promotor der Seq.-ID Nr. 2 (*AYNI1*) anstelle Seq.-ID Nr. 1 hergestellt. Dazu wurde per PCR ein 611 bp Fragment amplifiziert, das in 5' eine zusätzliche *Spe*I - Restriktionsschnittstelle und in 3' eine zusätzliche *Eco*RI-Restriktionsschnittstelle enthält.

Das entsprechende DNA-Konstrukt ist schematisch in Figur 4 gezeigt und weist in der erfindungsgemäβ verwendeten Expressionskassette das *Phytase K*-Gen unter der Kontrolle des Promotors der Seq.-ID Nr. 2 (*AYNI1*) auf, das in 3' vom *PHO5*-Terminator begrenzt wird. Zur Selektion ist wiederum eine Selektionsmarkerkassette mit der erfindungsgemäßen Expressionskassette verbunden, und beide Expressionskassetten sind beidseitig von Sequenzabschnitten flankiert, die zum Zielorganismus homolog sind, nämlich entsprechend der bevorzugten Ausführungsform homolog zur 25S rDNA. Zur Transformation wurde das linearisierte DNA-Konstrukt (YIEC) zwischen den *Nco*I-Restriktionsschnittstellen verwendet, sodass keine Sequenzabschnitte des E. *coli -* Shuttle-Vektors in den Zielorganismus eingebracht wurden.

Das erfindungsgemäße DNA-Konstrukt der Expressionskassette für *Phytase K* unter der Kontrolle des erfindungsgemäβ verwendeten Promotors der Seq.-ID Nr. 2 in Verbindung mit der Selektionsmarker-Expressionskassette *ATRP1* unter der Kontrolle des *ALEU2*-Promotors integrierte in *Arxula adeninivorans* unikal in die 25S rDNA.

Wie bereits für den Promotor von Seq.-ID Nr. 1 in Beispiel 1 gezeigt, war auch der Promotor der Seq.-ID Nr. 2 durch NO₃⁻ oder NO₂⁻ induzierbar, während in G-HMM mit NH₄⁺ keine Aktivität gemessen wurde, die von der erfindungsgemäß verwendeten Expressionskassette, hier am Beispiel der *Phytase K,* translatiert wurde.

Diese Induktion durch NO₃⁻ bzw. NO₂⁻ im G-HMM bei fehlende Aktivität des Promotors in Gegenwart von NH₄⁺ zeigt, dass die erfindungsgemäß verwendete Promotoren vorzugsweise durch NO₃⁻ bzw. NO₂⁻ induzierbar sind, während in Abwesenheit von NO₃⁻ bzw. NO₂⁻ keine Induktion erfolgt, und/oder die erfindungsgemäß verwendeten Promotoren vorzugsweise durch NH₄⁺ im G-HMM reprimiert werden.

### Beispiel 3: Promotor auf Basis der Seq.-ID Nr. 3

Entsprechend Beispiel 2 wurden Expressionskassetten hergestellt, jedoch mit dem Promotor der Seq.-ID Nr. 3 (*AYNT1*) anstelle Seq.-ID Nr. 2.

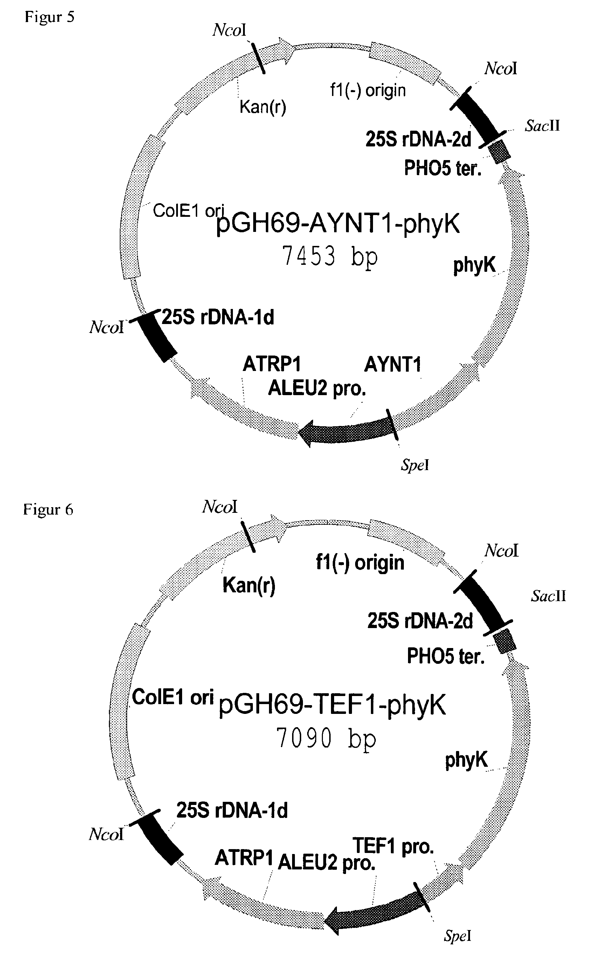

Das entsprechende DNA-Konstrukt ist schematisch in Figur 5 gezeigt und weist in der erfindungsgemäß verwendeten Expressionskassette das *Phytase K*-Gen unter der Kontrolle des Promotors der Seq.-ID Nr. 3 (*AYNT1*) auf, das in 3' vom *PHO5*-Terminator begrenzt wird. Zur Selektion ist wiederum eine Selektionsmarkerkassette mit der erfindungsgemäßen Expressionskassette verbunden, und beide Expressionskassetten sind beidseitig von Sequenzabschnitten flankiert, die zum Zielorganismus homolog sind, nämlich entsprechend der bevorzugten Ausführungsform homolog zur 25S rDNA. Zur Transformation wurde das linearisierte DNA-Konstrukt (YIEC) zwischen den NcoI-Restriktionsschnittstellen verwendet, sodass keine Sequenzabschnitte des *E*. *coli -* Shuttle-Vektors in den Zielorganismus eingebracht wurden.

Wie bereits für den Promotor von Seq.-ID Nr. 2 in Beispiel 2 gezeigt, war auch der Promotor der Seq.-ID Nr. 3 durch NO₃⁻ oder NO₂⁻ induzierbar, während in dem G-HMM mit NH₄⁺ keine Aktivität gemessen wurde, die von der erfindungsgemäßen Expressionskassette, hier am Beispiel der Phytase K, translatiert wurde.

### Beispiel 4: Promotoren auf Basis der Seq.-ID Nr.4. Seq.-ID Nr.5 bzw. Seq.-ID Nr.6

Entsprechend den Beispielen 1-3 wurden Expressionskassetten hergestellt, jedoch mit den auf 50% verkürzten Promotoren der Seq.-ID Nrn. 4 (*AYNR1*), 5 (*AYNI1'*) bzw. 6 (*AYNT1'*) anstelle der vollständigen Promotoren mit Seq.-ID Nrn. 1, 2 bzw. 3.

Die entsprechenden DNA-Konstrukte weisen in den erfindungsgemäß verwendeten Expressionskassetten das *Phytase K*-Gen unter der Kontrolle der Promotoren der Seq.-ID Nrn. 4 (*AYNR1'*), 5 (*AYNI1'*) bzw. 6 (*AYNT1'*) auf, die in 3' vom *PHO5*-Terminator begrenzt werden. Zur Selektion ist wiederum eine Selektionsmarkerkassette mit der erfindungsgemäß verwendeten Expressionskassette verbunden, und beide Expressionskassetten sind beidseitig von Sequenzabschnitten flankiert, die zum Zielorganismus homolog sind, nämlich entsprechend der bevorzugten Ausführungsform homolog zur 25S rDNA. Zur Transformation wurde das linearisierte DNA-Konstrukt (YIEC) zwischen den *Nco*I-Restriktionsschnittstellen verwendet, sodass keine Sequenzabschnitte des *E*. *coli -* Shuttle-Vektors in den Zielorganismus eingebracht wurden.

Wie bereits für die Promotoren von Seq.-ID Nrn. 1-3 in den Beispielen 1-3 gezeigt, sind auch die gegenüber den Promotoren der Seq.-ID Nrn. 1 -3 verkürzten Promotoren der Seq.-ID Nrn. 4-6 durch NO₃⁻ oder NO₂⁻ induzierbar, während in dem G-HMM mit NH₄⁺ keine Aktivität gemessen wurde, die von der erfindungsgemäßen Expressionskassette, hier am Beispiel der Phytase K, translatiert wurde.

Bei Ersetzen des Promotors der Seq.-ID Nr. 4 durch die Sequenz Seq.-ID Nr. 8 wurden ähnliche Ergebnisse erzielt.

### Beispiel 5: Vergleich der Aktivitäten von Promotoren mit Seq.-ID Nr. 1, Seq.-ID Nr. 2 und Seq.-ID Nr. 3

Zur Einschätzung der Promotoraktivität der erfindungsgemäß verwendeten Promotoren wurde ein Vergleich der Translationsraten, bestimmt als Aktivität der Phytase K, für die erfindungsgemäß verwendeten Expressionskassetten von Beispielen 1, 2 und 3 mit einem Vergleichs-Transformanden verglichen, der anstelle eines erfindungsgemäßen Promotors den *TEF1 -* Promotor in 5' vor dem *Phytase K*-Gen enthielt, in einem ansonsten identischen DNA-Konstrukt mit der selben Selektionsmarkerkassette. Das Vergleichs-Konstrukt ist schematisch in Figur 6 gezeigt. Auch der Vergleichs-Transformand enthielt eine unikal ins Genom inserierte Expressionskassette für Phytase K, nämlich unter der Kontrolle des *TEF1-*Promotors.

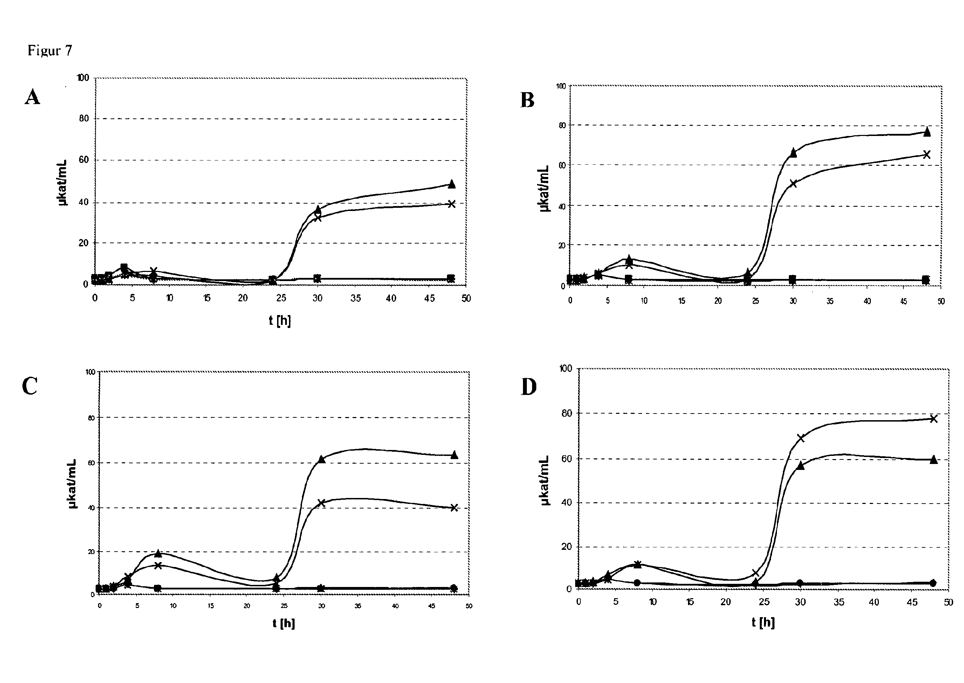

Der *TEF1*-Promotor ist stark konstitutiv. Die Transformanden wurde nach anfänglicher Kultivierung in G-HMM mit NH₄⁺ für 48 h bei 30 °C kultiviert, geerntet und direkt in frisches G-HMM mit NH₄⁺ (■, ◆) bzw. NO₃⁻ (x, ▲) übertragen und für weitere 48 h bei 30 °C kultiviert. Dabei sind in Figur 7 A die Ergebnisse für den Promotor mit Seq.-ID Nr. 1 gezeigt, in Figur 7 B für den Promotor Seq.-ID Nr. 2, in Figur 7 C für den Promotor Seq.-ID Nr. 3 und in Figur 7 D für den Promotor *TEF1*. Zum Vergleich ist jeweils die Negativ-Kontrolle G1212/YIEC69 (*, I) gezeigt. In G-HMM mit NH₄⁺ (■) zeigen die erfindungsgemäß verwendeten Promotoren keine Synthese von aktiver Phytase K.

Die extrazellulär gemessenen Phytase-Aktivitäten zeigen, dass der Promotor der Seq.-ID Nr. 2 etwa die Stärke des bisher bekannten stärksten konstitutiven Promotors *TEF1* erreicht, während die Promotoren Seq.-ID Nr. 1 und Seq.-ID Nr. 3 etwa zwei Drittel dieser Promotoraktivität aufweisen, wenn im G-HMM NO₃⁻ als Induktor vorliegt.

Dabei weisen die erfindungsgemäß verwendeten Promotoren den großen Vorteil auf, dass sie mit NO₃⁻ oder NO₂⁻ induzierbar, und in Abwesenheit von NO₃⁻ oder NO₂⁻ inaktiv sind, bzw. durch NH₄⁺ reprimiert werden.

### Beispiel 5: Expression von Enzymen, die bisher nicht von synthetischen Expressionskassetten in aktiver Form translatiert wurden

Als Beispiel für ein Translationsprodukt, das bisher nicht von synthetischen Expressionskassetten in enzymatisch aktiver Form translatiert wurde, wird für Tannase aus *Arxula adeninivorans* gezeigt, dass eine Expression enzymatish aktiven Translationsprodukts unter Kontrolle der erfindungsgemäß verwendeten Pomotoren möglich ist.

Bisher wurde die sekretorische Tannase aus *Arxula adeninivorans,* deren kodierende Sequenz als Seq.-ID Nrn. 3 und 4 in WO 2006/002955 beschrieben ist, mit dem Wildtyp von *Arxula* produziert.

Bisherige Versuche der Erfinden, Tannase entsprechend des Wildtyps von *Arxula* mit Tannin bzw. Gallussäure als C-Quelle auch als rekombinantes Enzym unter Nutzung von Glukose als C-Quelle zu synthetisieren, führten nicht zum Erfolg. Auch eine Expressionskassette mit dem *TEF1*-Promotor, funktional vor dem *Tannase*-Gen (*ATAN*) angeordnet und mit einem *PHO5-*Terminator in 3' zum *ATAN*-Gen, ergaben bei Kultivierung in G-HMM nur sehr geringe Konzentrationen an rekombinanter Tannase. Auch mehrfache Integrationen der *ATAN-*Expressionskassette mit den nativen *ATAN*-Promotor führte nur dann zur Synthese enzymatisch aktiver Tannase, wenn Gallussäure bzw. Tannin als C-Quelle eingesetzt wurden, und nur in Konzentrationen entsprechend des Wildtyps.

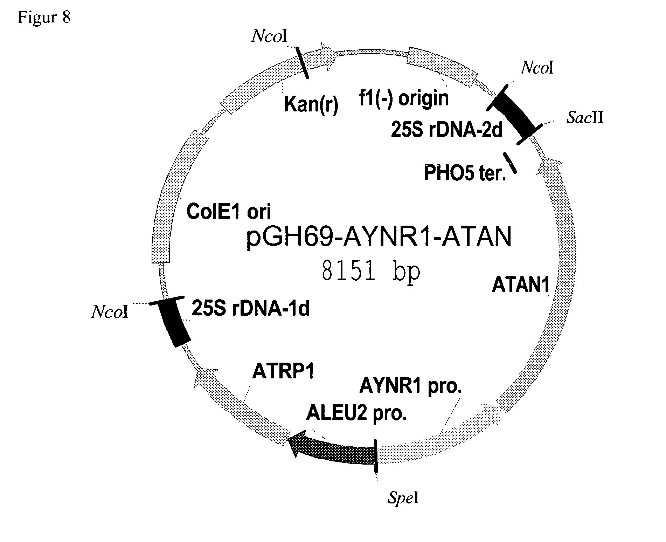

Zur Expression der Tannase in genetisch manipulierter *Arxula* wurde eine Expressionskassette kloniert, in der das *Tannase*-Gen (*ATAN*) in 5' von einem erfindungsgemäß verwendeten Promotor, hier am Beispiel der Seq.-ID Nr. 1, und in 3' vom *PHO5-*Terminator flankiert ist. Diese Expressionskassette für Tannase ist mit einer Selektionsmarker-Expressionskassette (*ATRP1*) verbunden und beidseitig flankiert von homologen Sequenzen, nämlich Abschnitten der 25S rDNA. Das DNA-Konstrukt ist schematisch in Figur 8 in einem *E. coli* Shuttle-Vektor gezeigt. Zur Transformation wurden die miteinander verbundenen *ATAN -* und die Selektionsmarker-Expressionskassetten einschließlich der flankierenden Abschnitte der 25S rDNA (YIEC) nach Restriktion mit *Nco*I verwendet. Nach integrativer Transformation von *Arxula adeninivorans* mit Auxotrophiemarker Trp⁻ (G 1212 [*aleu2 ALEU2::Δatrp1*] (Steinborn G, Wartmann T, Gellissen G, Kunze G (2007) J. Biotechnol. 127: 392-401) als Zielorganismus entsprechend Beispiel 1 konnte Tannase in NO₃⁻-haltigem Medium exprimiert werden. Im Anschluss an die Transformation wurde zunächst in Vollmedium, anschließend zur Selektion in G-HMM kultiviert. Erhaltene Transformanden weisen eine unikal in die 25S rDNA integrierte Kopie der erfindungsgemäß verwendeten Expressionskassette auf.

Zur Expression von Tannase wurde ein Transformand bis zu 97 h in G-HMM mit NO₃⁻ als Stickstoffquelle kultiviert, als Vergleich ein Kontrollstamm G1212/YIEC69 ohne rekombinantes *Tannase*-Gen eingesetzt.

Sowohl erfindungsgemäß verwendete Transformanden mit *Tannase*-Gen, als auch der Kontrollklon weisen dieselben Wachstumsraten mit NH₄⁺ bzw. NO₃⁻ als Stickstoffquelle auf. Nur Transformanden mit erfindungsgeäß verwendeter Expressionskassette fiir Tannase akkumulieren während des Wachstums in Medium mit NO₃⁻ oder NO₂⁻ rekombinante Tannase. Die Konzentration der Tannaseaktivität steigt während der Kultivierung kontinuierlich an und erreicht in der stationären Wachstumsphase, nach ca. 67 h das Maximum. Während der anschließenden Kultivierung bis insgesamt 97 h war die Aktivität der rekombinanten Tannase nahezu konstant.

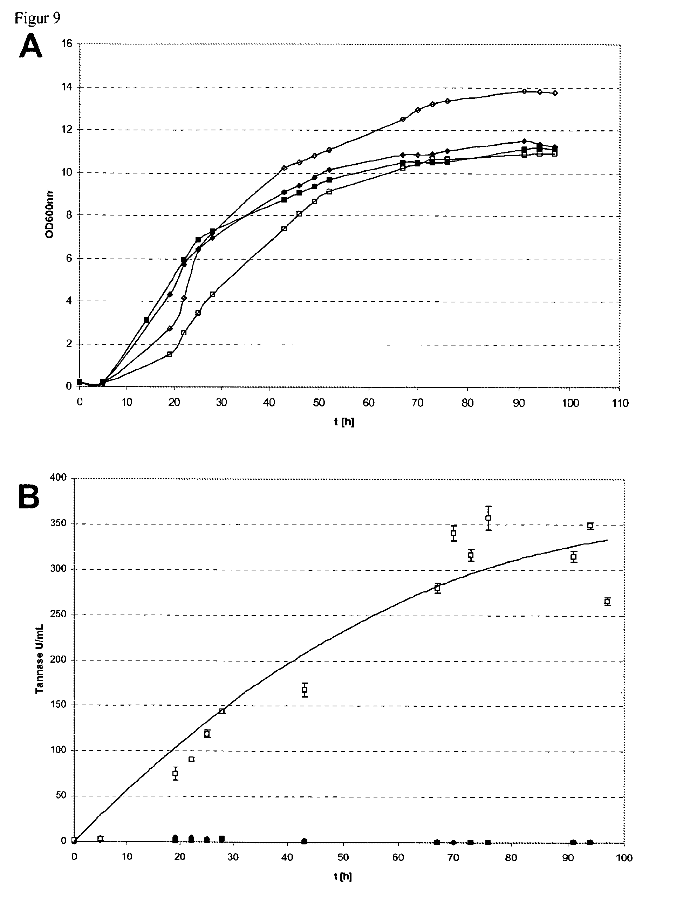

Die Wachstumsrate ist anhand der optischen Dichte in Figur 9 A gezeigt, die Aktivität der Tannase in Figur 9 B, für die Kontrolle G1212/YIEC69 (◆ in NH₄⁺, 0 in NO₃⁻) bzw. die erfindungsgemäßen Transformanden in NH₄⁺ (■), bzw. in NO₃⁻ (□).

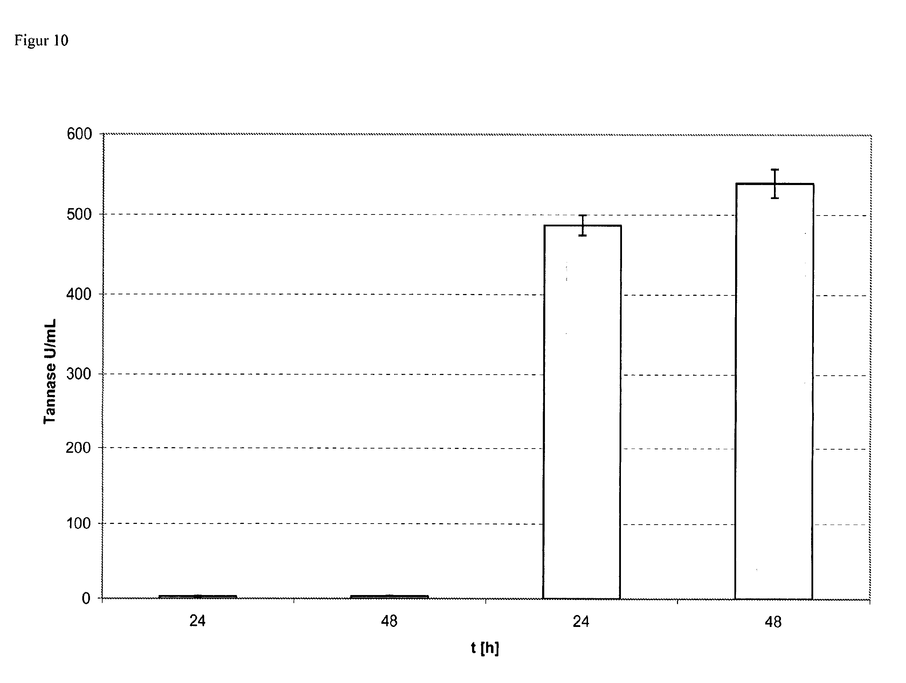

Die Induktion der Transformanden mit *Tannase-Gen* konnte auch im sogenannten Shift-Experiment, d.h. nach Kultivierung unter nicht-induzierenden Bedingungen im NO₃⁻- und NO₂⁻-freien G-HMM und anschließende Übertragung in G-HMM mit NO₃⁻ oder NO₂⁻ erzielt werden. So konnten die Transformanden mit *Tannase-Gen* und Vergleichs-Transformanden nach Kultivierung in G-HMM mit NH₄⁺ über 48 h, während derer keine Tannase produziert wurde, abzentrifugiert werden und in frisches G-HMM mit NH₄⁺ bzw. NO₃⁻ übertragen werden. Nur der Transformand mit *Tannase*-Gen (G1212/YIEC69-AYNR1-ATAN) produzierte im NO₃⁻-haltigen Medium rekombinante Tannase, die extrazellulär nachgewiesen wurde. Im Anschluss an die Übertragung in NO₃⁻- bzw. NO₂⁻-haltiges Medium stieg die Aktivität der Tannase kontinuierlich an und erreichte nach etwa 24-48 h ihr Maximum; die Messung der Tannaseaktivität des erfindungsgemäß verwendeten Transformanden ist in Figur 10 gezeigt, nämlich links nach 24 h bzw. 48 h in NH₄⁺-haltigem Medium und rechts nach 24 h bzw. 48 h in NO₃⁻ -haltigem Medium. Während der weiteren Kultivierung blieb die Aktivität der Tannase nahezu konstant, was zeigt, dass zumindest die einmal erreichte Konzentration enzymatisch aktiven Translationsprodukts der erfindungsgemäßen Expressionskassette nicht degradiert wird. Im Gegensatz dazu konnte beim Kontrollstamm *A. adeninivorans* G1212/YIEC69 auch im NO₃⁻haltigen Medium weder intra- noch extrazellulär eine Tannaseaktivität gemessen werden.

## Patentansprüche

1. Verfahren zur intrazellulären oder extrazellulären Proteinexpression in Arxula mit einer Expressionskassette mit einem Strukturgen, das funktional zu einem Promotor angeordnet ist, **dadurch gekennzeichnet, dass** die das zu exprimierende Protein kodierende DNA-Sequenz unter der Kontrolle einer DNA- Sequenz mit Promotoraktivität angeordnet ist, die durch Nitrat oder Nitrit induzierbar ist und eine der Sequenzen Seq.-ID Nr. 1, Seq.-ID Nr. 2 oder Seq.-ID Nr. 3 aufweist.

2. Verfahren nach Anspruch 1, **dadurch gekennzeichnet, dass** die DNA- Sequenz mit Promotoraktivität einen Abschnitt mit einer Länge von mindestens 50% einer der Sequenzen Seq.-ID Nr. 1, Seq.-ID Nr. 2 oder Seq.-ID Nr. 3 aufweist und eine Sequenz der Seq.-ID Nrn. 4, 5 oder 6 hat.

3. Verfahren nach Anspruch 1 oder 2, **dadurch gekennzeichnet, dass** die Pilzzellen durch Transformation mit einem Vektor erzeugt werden, der die kodierende DNA-Sequenz und die DNA- Sequenz mit Promotoraktivität enthält.

4. Verfahren nach Anspruch 3, **dadurch gekennzeichnet, dass** der Vektor in das Genom der Zelle integriert wird.

5. Verfahren nach einem der voranstehenden Ansprüche, **dadurch gekennzeichnet, dass** das zu exprimierende Protein eine enzymatische Aktivität hat.

6. Verfahren nach Anspruch 5, **dadurch gekennzeichnet, dass** die enzymatische Aktivität eine Tannaseaktivität ist.

7. Verfahren nach Anspruch 1, **dadurch gekennzeichnet, dass** die DNA- Sequenz mit Promotoraktivität ein Derivat ist, das Sequenzabweichungen von maximal 5% von der Sequenzen Seq.-ID Nr. 1, Seq.-ID Nr. 2 oder Seq.-ID Nr. 3 aufweist.

## Claims

1. Process for intracellular or extracellular protein expression in Arxula from an expression cassette having a structural gene arranged functionally to a promoter, **characterized in that** the DNA sequence encoding the protein to be expressed is arranged under the control of a DNA sequence having promoter activity which is inducible by nitrate or nitrite and has one of the sequences SEQ ID NO: 1, SEQ ID NO: 2 or SEQ ID NO: 3.

2. Process according to claim 1, **characterized in that** the DNA sequence having promoter activity has a section having a length of at least 50% of one of the sequences of SEQ ID NO: 1, SEQ ID NO: 2 or SEQ ID NO: 3 and a sequence of SEQ ID NO: 4, 5 or 6.

3. Process according to claim 1 or 2, **characterized in that** the fungal cells are generated by transformation with a vector containing the coding DNA sequence and the DNA sequence having promoter activity.

4. Process according to claim 3, **characterized in that** the vector is integrated into the genome of the cell.

5. Process according to one of the preceding claims, **characterized in that** the protein to be expressed has an enzymatic activity.

6. Process according to claim 5, **characterized in that** the enzymatic activity is a tannase activity.

7. Process according to claim 1, **characterized in that** the DNA sequence having promoter activity is a derivative having sequence deviations of at maximum 5% from the sequences SEQ ID NO: 1, SEQ ID NO: 2 or SEQ ID NO: 3.

## Revendications

1. Procédé d'expression intracellulaire ou extracellulaire de protéines dans Arxula à l'aide d'une cassette d'expression munie d'un gène de structure qui est disposé fonctionnellement par rapport à un promoteur, **caractérisé en ce que** la séquence d'ADN codant pour la protéine à exprimer est placée sous le contrôle d'une séquence d'ADN, à activité de promoteur, qui peut être induite par nitrate ou par nitrite et qui comporte une des séquences SEQ ID n° 1, SEQ ID n° 2 ou SEQ ID n° 3.

2. Procédé selon la revendication 1, **caractérisé en ce que** la séquence d'ADN à activité de promoteur comporte un segment dont la longueur représente au moins 50 % de celle d'une des séquences SEQ ID n° 1, SEQ ID n° 2 ou SEQ ID n° 3 et possède une séquence des SEQ ID n° 4, n° 5 ou n° 6.

3. Procédé selon la revendication 1 ou 2, **caractérisé en ce que** les cellules fongiques sont produites par transformation avec un vecteur qui contient la séquence d'ADN codante et la séquence d'ADN à activité de promoteur.

4. Procédé selon la revendication 3, **caractérisé en ce que** le vecteur est intégré dans le génome de la cellule.

5. Procédé selon l'une des revendications précédentes, **caractérisé en ce que** la protéine à exprimer a une activité enzymatique.

6. Procédé selon la revendication 5, **caractérisé en ce que** l'activité enzymatique est une activité de tannase.

7. Procédé selon la revendication 1, **caractérisé en ce que** la séquence d'ADN à activité de promoteur est un dérivé qui comporte des différences de séquence qui représentent au maximum 5 % des séquences SEQ ID n° 1, SEQ ID n° 2 ou SEQ ID n° 3.


### SEQUENCE LISTING

<110> Leibniz-Institut für Pflanzengenetik und Kulturpflanzenforschung
<120> Eukaryotische Promotoren zur Genexpression
<130> I1004PCT
<150> DE 10 2007 038 037.4
   <151> 2007-08-10
<160> 8
<170> PatentIn version 3.4
<210> 1
   <211> 885
   <212> DNA
   <213> Arxula adeninivorans: Seq.-ID Nr. 1
<400> 1
   agcagtgatt tagcagcttc tagtcccagc agaccaccgc cgacgacaat ggcgcgcttt      60
   cctttagtct caaacttgtc ggtagaaaag cgaatcattt cctgaagatc atccactgtg     120
   cgatagacaa aaattccatg cgaacgagat tcctccacgg atggctcgta tccaagggac     180
   cgagagatga tgtccgatgg tagaactgcc cggcttcctg tagccatgat taggagatca     240
   tagctatatt cgtgccccaa gttagtcaaa accttcttgg tcttagtatc aatgctagtg     300
   acgggctcgt taactcggca caccatatgg tgtggatcta tgctttcata ccaggcagtg     360
   tcattgagat agaggtcatt gactgactgg tgcgaaaagt agtcggccaa tcccactcgg     420
   ttgtaggcca aatacttctc ttctcctaga atggtaacgg aatattctct ggttaataga     480
   tccctttcca gaagctgttc cacaaacgac atgccaacca ttccaaagcc caccaggaca     540
   atttgctttg gcggagtggc caatccgttg tgtgttattg tcatggatcc tgtaagtgtg     600
   tagtcaagca attgcaaaac gttgaccagg atgatccaag taataaatta ctttaccctt     660
   atcatgctta tcgtccactc gggtggcctc ggggtttttc gaaccgaact gataagatag     720
   tgccccattt tcgggacgaa agacgccgat ccgagataag acccctcgga acatggtgat     780
   aagtaatcct cgcaaataga ttggacagtt tgacaatgat tagagttttg gagaatatat     840
   agacgttatc agtacatggg gggggatagt tggcattgac tattg                     885
<210> 2
   <211> 599
   <212> DNA
   <213> Arxula adeninivorans: Seq.-ID Nr. 2
<400> 2
   tctcaaccat gccatcaatc tcaatctccc agtccagcac gtcgttcgta gattcaggta      60
   ccggtccatg gttacggaca aagtggagcg acgagggtgt aatgaatcca gcgttgtaca     120
   acaagtccaa aggtgcctca gaattaaacg gatgagctcc agtcaacctc acgagccgtt     180
   cgtcccgagg aacatgcgag tctggtgtct tcttgtcgag ttccagcaca tgttcagctt     240
   tggacggtcc ttgaaacgct ggcccggtca gaattttttc aactgtttgg tattctggtg      300
   ccatcaatag tcaatgccaa ctatcccccc ccatgtactg ataacgtcta tatattctcc 360
   aaaactctaa tcattgtcaa actgtccaat ctatttgcga ggattactta tcaccatgtt 420
   ccgaggggtc ttatctcgga tcggcgtctt tcgtcccgaa aatggggcac tatcttatca 480
   gttcggttcg aaaaaccccg aggccacccg agtggacgat aagcatgata agggtaaagt 540
   aatttattac ttggatcatc ctggtcaacg ttttgcaatt gcttgactac acacttaca 599
<210> 3
   <211> 663
   <212> DNA
   <213> Arxula adeninivorans: Seq.-ID Nr. 3
<400> 3
   cttcggaaac agatatgagg atgatatttt atgcaaaagc gaattgtgca gatttgaaca 60
   aatgacgcag gggcggatcc ggataaagct ctggctatct ggcaaaatgg accaatgcaa 120
   tactcagcat actcccggaa ggatcgatct gcaaataatg gatcgtgagc tgtcgcacat 180
   gaaggagggt cacgattcaa tgctgctagt atgtggacca gaaggaatgg tggagactgc 240
   taaaacatgg caaaagtcca ggaacattga agattttagg gtagtctatt tttaagcata 300
   taacagatat tttaagaaaa ttggcatagc tctacttcac ttttgtaacg acagctcgga 360
   tcctggtaat accgagtttt aaagaaaaat gaattgttca catggaccat gcagtatctg 420
   ataaagcata gataacgtta agtgagtgcg caccgtcctt aacctcggtt cgggtcaatg 480
   aaaaccgagc gaaagcggaa ttttcagacc gagacaccct tatcaggtcg ttatcagaac 540
   cttatctcga tcttatcagc tataacgctc agcggggtcg tatgcaaggg acaggtttaa 600
   ataatgtctc ggaattggca tataataaat accgttgaaa ctttcataac tacagcatag 660
   aca 663
<210> 4
   <211> 465
   <212> DNA
   <213> Arxula adeninivorans: Seq.-ID Nr. 4
<400> 4
   ttgtaggcca aatacttctc ttctcctaga atggtaacgg aatattctct ggttaataga 60
   tccctttcca gaagctgttc cacaaacgac atgccaacca ttccaaagcc caccaggaca 120
   atttgctttg gcggagtggc caatccgttg tgtgttattg tcatggatcc tgtaagtgtg 180
   tagtcaagca attgcaaaac gttgaccagg atgatccaag taataaatta ctttacccct 240
   atcatgctta tcgtccactc gggtggcctc ggggtttttc gaaccgaact gataagatag 300
   tgccccattt tcgggacgaa agacgccgat ccgagataag acccctcgga acatggtgat 360
   aagtaatcct cgcaaataga ttggacagtt tgacaatgat tagagttttg gagaatatat 420
   agacgttatc agtacatggg gggggatagt tggcattgac tattg 465
<210> 5
   <211> 299
   <212> DNA
   <213> Arxula adeninivorans: Seq.-ID Nr. 5
<400> 5
   ccatcaatag tcaatgccaa ctatcccccc ccatgtactg ataacgtcta tatattctcc 60
   aaaactctaa tcattgtcaa actgtccaat ctatttgcga ggattactta tcaccatgtt 120
   ccgaggggtc ttatctcgga tcggcgtctt tcgtcccgaa aatggggcac tatcttatca 180
   gttcggttcg aaaaaccccg aggccacccg agtggacgat aagcatgata agggtaaagt 240
   aatttattac ttggatcatc ctggtcaacg ttttgcaatt gcttgactac acacttaca 299
<210> 6
   <211> 331
   <212> DNA
   <213> Arxula adeninivorans: Seq.-ID Nr. 6
<400> 6
   tacttcactt ttgtaacgac agctcggatc ctaccgagtt ttaaagaaaa atgaattgtt 60
   cacatggacc atgcagtatc tgataaagca ttggtaaaga taacgttaag tgagtgcgca 120
   ccgtccttaa cctcggttcg ggtcaatgaa aaccgagcga aagcggaatt ttcagaccga 180
   gacaccctta tcaggtcgtt atcagaacct tatctcgatc ttatcagcta taacgctcag 240
   cggggtcgta tgcaagggac aggtttaaat aatgtctcgg aattggcata taataaatac 300
   cgttgaaact ttcataacta cagcatagac a 331